# EUROPEAN PATENT SPECIFICATION

(11) **EP 2 687 598 B1**
(45) Date of publication and mention of the grant of the patent: **15.02.2017**
(21) Application number: 12758277.3
(22) Date of filing: 13.03.2012
(51) Int. Cl.: C12N 15/09

(54) **METHOD FOR ASSAYING ARYLSULFATASE ACTIVITY**
VERFAHREN ZUR ÜBERPRÜFUNG EINER ARYLSULFATASE-AKTIVITÄT
PROCÉDÉ POUR LE DOSAGE DE L'ACTIVITÉ ARYLSULFATASE

(30) Priority: 14.03.2011 JP 2011055259
(43) Date of publication of application: 22.01.2014
(73) Proprietor: Godo Shusei Co., Ltd., Tokyo, 1048162 (JP)
(72) Inventor: SHIOTA, Kazuma, Aomori 031-0072 (JP); HORIGUCHI, Hirofumi, Chiba 271-0064 (JP); IYOTANI, Ai, Chiba 271-0064 (JP); YOSHIKAWA, Jun, Chiba 271-0064 (JP); SATO, Tomoko, Tokyo 104-8162 (JP)
(74) Representative: Osha Liang
(86) International application number: PCT/JP2012/056340
(87) International publication number: WO 2012/124668

(56) References cited:
- WO-A1-02/081673
- WO-A1-2011/033633
- WO-A2-2007/060247
- LEE-VAUPEL M ET AL: "A SIMPLE CHROMOGENIC ASSAY FOR ARYLSULFATASE A", CLINICA CHIMICA ACTA, vol. 164, no. 2, 1987, pages 171-180, XP002728353, ISSN: 0009-8981
- FRESE MARC-ANDRE ET AL: "Arylsulfatase G, a novel lysosomal sulfatase", JOURNAL OF BIOLOGICAL CHEMISTRY, AMERICAN SOCIETY FOR BIOCHEMISTRY AND MOLECULAR BIOLOGY, US, vol. 283, no. 17, 1 April 2008 (2008-04-01), pages 11388-11395, XP002506677, ISSN: 0021-9258, DOI: 10.1074/JBC.M709917200 [retrieved on 2008-02-18]
- STEVENS R.L.: 'Minor anionic arylsulfatases in cultured human fibroblasts' BIOCHIM. BIOPHYS. ACTA vol. 370, no. 1, 25 November 1974, pages 249 - 256, XP023670301
- DAE-SEOK B. ET AL.: 'Isolation and characterization of marine bacterium producing arylsulfatase' JOURNAL OF MICROBIOLOGY AND BIOTECHNOLOGY vol. 14, no. 6, 2004, pages 1134 - 1141, XP055125336
- HUSSEIN L. ET AL.: 'Reduction of lactose in milk by purified lactase produced by Kluyveromyces lactis' JOURNAL OF FOOD PROTECTION vol. 52, no. 1, January 1989, pages 30 - 34, XP003028138
- KIM C.S. ET AL.: 'Expression and characterization of Kluyveromyces lactis P- galactosidase in Escherichia coli' BIOTECHNOL. LETT. vol. 25, no. 20, October 2003, pages 1769 - 1774, XP002378207
- BECERRA M. ET AL.: 'Micro-scale purification of P-galactosidase from Kluyveromyces lactis reveals that dimeric and tetrameric forms are active' BIOTECHNOLOGY TECHNIQUES vol. 12, no. 3, March 1998, pages 253 - 256, XP002378514
- INOUE H. ET AL.: 'Fluorometric determination of arylsulfatase A and B activities' CHEM. PHARM. BULL. vol. 30, no. 11, November 1982, pages 4140 - 4143, XP055131831

### SEQUENCE LISTING

<110> GODO SHUSEI CO., LTD.
<120> Process for Determining Activity of Arylsulfatase
<130> GS11001
<160> 6
<170> PatentIn version 3.1
<210> 1
   <211> 22
   <212> DNA
   <213> Kluyveromyces lactis
<400> 1
   atgaccaaaa cagatgaacc ta 22
<210> 2
   <211> 19
   <212> DNA
   <213> Kluyveromyces lactis
<400> 2
   ccagtctctt gcgtcgtga 19
<210> 3
   <211> 28
   <212> DNA
   <213> Kluyveromyces lactis
<400> 3
   gaagatctca gacctgaggt aacgtttc 28
<210> 4
   <211> 20
   <212> DNA
   <213> Kluyveromyces lactis
<400> 4
   ctgggtaact tgttctggtg 20
<210> 5
   <211> 59
   <212> DNA
   <213> Kluyveromyces lactis
<400> 5
   atcgatgttg ttgagcggta ctgacaacca tttggcaggt ttcatcaaca cacgagccg 59
<210> 6
   <211> 53
   <212> DNA
   <213> Kluyveromyces
<400> 6
   atcgataact ctaccgatat tttggtctaa ttcatcaacc actgttggtg gag 53

## Description

### TECHNICAL FIELD

The present invention relates to a high sensitive method for determining activity of arylsulfatase, a lactase preparation in which it has been confirmed that arylsulfatase does not contaminate the preparation or exists in a small amount if it contaminates the preparation by the high sensitive method of arylsulfatase according to the present invention, a method for producing such a lactase preparation, and a dairy product that has been produced by using such a lactase preparation.

### BACKGROUND ART

Since ancient times, cow milk has been applied as a nutritious and useful food over the long term. Cow milk comprises lactose that is one of sugar. The lactose is decomposed by lactase in intestine. However, because in part of humans secretion volume of lactase into intestine decreases with growth, the part of humans develop so-called lactose intolerance with symptoms of abdominal pain and diarrhea if they takes a large amount of cow milk or its processed product (hereafter collectively-referred to "a dairy product"). This has been one reason by which a wide intake of this nutritious food is prohibited.

In recent years, dairy products from which lactose is previously reduced or removed are provided. The humans suffering from lactose intolerance can also intake such a dairy product without any problems.

The reduction or removal of lactose is performed by various methods, and the most common method is one in which lactose is hydrolyzed by treating a dairy product with a lactase preparation.

Previously, dairy products that had been obtained by decomposing lactose with lactase were commonly distributed after a sterilization step. However, recently, a means is prevalent, in which means a lactase preparation is added to sterile cow milk under aseptic conditions and lactose is decomposed during distribution. There are thought that by this means the amount of the lactase preparation to be used can be reduced and that this means contributes to cost reduction.

On the other hand, by employing the means in which a lactase preparation is added to sterile cow milk under aseptic conditions, a new problem has been arisen. It is a problem, of which cause is that contaminated enzymes (protease and arylsulfatase) that are contained in a small amount in the lactase preparation cause changes to milk constituents because those enzymes have not been inactivated. In fact, it has been known that protease causes curd or development of bitter taste. Further, in Non-patent Literature 1, it is reported that arylsulfatase causes developments of unreasonable and undesirable taste and smell.

Patent Literature 1 describes a lactase preparation in which the amount of contaminated arylsulfatase is reduced and a method for producing it. However, by the method for determining activity of arylsulfatase that is described in Patent Literature 1, arylsulfatase activity cannot be determined in a range of trace amounts of 8 units or less (arylsulfatase activity per 1 NLU of a substance having lactase activity, hereafter the same shall be applied), and it is only described as a detection limit or less. Further, Table 1 of Patent Literature 1 describes that if the activity of the contaminated arylsulfatase is 19 units or less, off-flavor will not occur in the dairy product. However, this judgment is based on the result of a short-term examination in which the reaction term is 2 days. It is inferred that the cause, by which off-flavor develops in dairy products, is a chemical change of a milk constituent by an enzyme, arylsulfatase, and a long reaction term of 2 to 3 months or more is assumed in shelf-stable milk at ordinary temperatures (UHT milk) as its use. If so, it cannot be said that a lactase preparation having an arylsulfatase activity of 19 units or less, which has been determined by the method according to Patent Literature 1, will not develop off-flavor in dairy products. They go without saying that it is necessary to control more rigorously the amount of the contaminated arylsulfatase and that it is necessary to employ a higher sensitive method for determining activity of arylsulfatase to control the amount of the contaminated arylsulfatase.

To eliminate the arylsulfatase that contaminates an enzyme preparation to be the minimum quantity, we should perform purification by combining general purification methods. Alternatively, we should select a microoraganism that can produce an intended enzyme, of which arylsulfatase producibility has been deleted, and culture the microoraganism. Also, we can use a microorganism which has been obtained by transforming a host that intrinsically produces no arylsulfatase to have it producing an intended enzyme.

Patent Literature 1 includes descriptions about a process for obtaining a microorganism, of which arylsulfatase producibility has been reduced by mutation treatments, and a microorganism, of which arylsulfatase gene has been deleted by genetic engineering procedures. However, although the process for obtaining a microorganism, of which arylsulfatase producibility has been reduced by mutation treatments, is described, there is no example in which the microorganism has been actually obtained. Namely, only a possibility for it is shown. In other words, it is unexplained whether the microorganism, of which arylsulfatase producibility has been reduced by mutation treatments, can be produced in an industrial scale according to the process as described in Patent Literature 1. To disrupt arylsulfatase genes by mutation in a diploid strain of yeast that is useful for producing a lactase preparation in an industrial scale, it is necessary to obtain a double mutant. The obtaining of such a double mutant has been thought to be difficult in practice.

About the deletion of the arylsulfatase gene by genetic engineering procedures, only an example of CBS2359 strain that is a monoploid is described in Patent Literature 1. In other words, by the process as described in Patent Literature 1, it is difficult to effectively disrupt genes in a diploid strain of yeast that is useful for producing a lactase preparation, and thus a strain that does not produce arylsulfatase and that is a diploid strain of yeast cannot be prepared.

About the lactase activity of the lactase preparations that are described in examples of Patent Literature 1, it is about 5,000 to 5,500 NLU/g for Maxilact (registered trademark) LG5000 (produced by DSM) and about 5,000 to 5,500 NLU/g for GODO YNL2 (produced by Godo Shusei Co., Ltd.). Further, the lactase activity of the lactase preparation is unclear, of which preparation has been produced from lactase that has been produced by a microorganism, of which arylsulfatase producibility has been reduced by the mutation treatments that are described in examples of Patent Literature 1. However, if the amount of the lactase preparation that is added to a dairy product is increased to obtain a sufficient lactase activity, as a matter of course, the absolute amount of arylsulfatase that contaminates the lactase preparation will increase. Therefore, the potential of development of off-flavor will be enhanced in dairy products such as shelf-stable milk at ordinary temperatures (UHT milk).

### PRIOR-ART LITERATURES

### PATENT LITERATURES

Patent Literature 1: Japanese Patent Laid-open No. 2009-517061

### NON-PATENT LITERATURES

Non-patent Literature 1: V Lopez, R. C., J. Agric. Food Chem. (1993), 41, p.p. 446-454

### SUMMARY OF INVENTION

### PROBLEMS TO BE SOLVED BY INVENTION

Even if the amount of arylsulfatase that contaminates a lactase preparation is a tiny amount, depending on the term of action, a temperature condition, or the like unreasonable and undesirable taste and smell can be developed when the lactase preparation is used by adding it to milk or a dairy product. Not to cause such a disadvantage, a lactase preparation having a higher lactase activity is desired, in which preparation the amount of arylsulfatase contaminated is reduced to be a minimum quantity, and preferably the arylsulfatase is completely eliminated. For this purpose, it is necessary to develop a method for determining activity of arylsulfatase, by which method, it can be confirmed that the amount of the arylsulfatase contaminated in the lactase preparation is reduced to be a minimum quantity.

### MEANS FOR SOLVING PROBLEM

The present inventors have extensively studied to solve the above problem and have developed a method for determining activity of arylsulfatase in an aqueous system having a sensitivity higher than that of a conventional method. Further, by the method for determining activity of arylsulfatase having a sensitivity higher than that of the conventional method, which is a fluorescence one, they have determined the activity of arylsulfatase that has contaminated a lactase preparation in a region where it has conventionally considered as to be equal to or less than the detection limit, and have specified the contaminated amount of the arylsulfatase, at which amount it will not develop undesirable taste or smell in milk or dairy products. Thus, they have accomplished the present invention.

Namely, the present invention relates to a method for determining activity of arylsulfatase in an aqueous system characterized in that arylsulfatase is subjected to reaction with a substrate, from which fluorophore or chromophore is liberated by suffering an action of the arylsulfatase, in an aqueous reaction system having high ionic strength.

Preferable examples of means for reaction in an aqueous reaction system having high ionic strength are one in which the enzyme is subjected to reaction with a substrate in an aqueous reaction system to which an inorganic salt has been added, and/or, another one in which the enzyme is subjected to reaction with a substrate in a buffer that does not denature the enzyme protein.

The preferable range of the concentration of the inorganic salt in the aqueous reaction system is 10 to 1000mM and more preferable range of it is 50 to 500mM, and the preferable range of the concentration of the buffer is 10 to 200mM and more preferable range of it is 50 to 200mM.

The above inorganic salt is preferably at least one member selected from the group consisting of potassium chloride, sodium chloride, and ammonium sulfate. Also, the above buffer is preferably a phosphate buffer.

The above method for determining activity of arylsulfatase in an aqueous system according to the present invention is particularly preferably one comprising the following steps (1) to (10):
(1) A specimen in which the existence of the arylsulfatase is predicted is arbitrarily diluted with 100mM potassium phosphate buffer (pH6.5) comprising 0.5M potassium chloride to obtain a sample.
(2) An aqueous solution comprising potassium 4-methylumbelliferone sulfate in a concentration of 2mM is prepared.
(3) The sample and the aqueous potassium 4-methylumbelliferone sulfate solution are mixed with each other at a ratio of 1:1 (volume basis) and are reacted at 37 degrees Celsius for 3 hours.
(4) To the reacted solution, 0.1N aqueous sodium hydroxide solution having the same amount (volume basis) as that of the reacted solution is added to stop the reaction, thus obtaining a sample for determination.
(5) Fluorescence intensity is determined at an excitation wavelength of 360nm and a fluorescence wavelength of 450nm.
(6) 4-Methylumbelliferone is dissolved in 100mM potassium phosphate buffer (pH6.5) comprising 0.5M potassium chloride to obtain a solution having an appropriate concentration, 0.1N aqueous sodium hydroxide solution is added in a similar way as that in step (4), and fluorescence intensity is determined under the same conditions as those in step (5).
(7) From step (6), a calibration curve is prepared.
(8) From the fluorescence intensity that was determined in step (5) and the calibration curve that was prepared in step (7), the concentration of 4-methylumbelliferone of the sample for determination is calculated, and the calculated value is divided by 3, thus obtaining the concentration of the 4-methylumbelliferone in the case where the reaction time of period is 1 hour. Further, from the volume of the reacted solution, the amount of the 4-methylumbelliferone that was liberated by the reaction of 1 hour is calculated.
(9) Because the amount of the 4-methylumbelliferone thus calculated is based on the amount of the specimen that was contained in the sample prepared in step (1), the calculated amount is converted to that of the 4-methylumbelliferone per 1g of the specimen.
(10) When the amount of the 4-methylumbelliferone that was liberated per 1 hour of the time of period of the reaction of the substrate and the enzyme is 1nmole, it is defined as 1 unit(U), and the unit is shown as a unit amount per 1g of the specimen (i.e., an enzyme preparation), namely, "unit(U)/g".

In step (10), the "substrate" is potassium 4-methylumbelliferone sulfate and the "enzyme" is arylsulfatase.

The above method for determining activity of arylsulfatase in an aqueous system according to the present invention can be applied for determining activity of arylsulfatase in a lactase preparation.

Further, the present invention relates to a lactase preparation produced by using, as a raw material, cultured yeast or microorganic cells and/or culture fluid of those cells, wherein the yeast or microorganic cells are those of a diploid strain of yeast having a lactase gene, in which expression of arylsulfatase protein is restricted, or a gene-recombinant microorganism in which a lactase gene of yeast has been transformed and expression of arylsulfatase protein is restricted, characterized in that the lactase preparation has a lactase activity of 4,000 NLU/g or more according to the FCC IV method and has an arylsulfatase activity of 0.1% or less of the lactase activity as the basis, in which the arylsulfatase activity (unit: U/g) has been determined and calculated by the method for determining activity of arylsulfatase in an aqueous system according to the present invention comprising the above steps (1) to (10).

The above lactase preparation according to the present invention can also be prepared without a step for removing arylsulfatase. Here, the "step for removing arylsulfatase" does not include such a step that arylsulfatase protein is purified with lactase protein as an intended enzyme, such as, e.g., ammonium sulfate fractionation from an aqueous solution comprising an intended enzyme, among fractionation and purification methods that are performed in this technical field. The step is one by which the lactase protein as the intended enzyme is separated from the arylsulfatase protein.

In the above present invention, "expression of arylsulfatase protein is restricted" means that arylsulfatase protein is not produced or its production amount is reduced because, e.g., an arylsulfatase gene (structural gene) has been disrupted, an expression regulation gene that works the arylsulfatase gene to express arylsulfatase protein has been disrupted, or there is no arylsulfatase gene and/or no expression regulation gene for the arylsulfatase protein. It is preferable that there is no expression of arylsulfatase protein, i.e., its production amount is zero. However, it is acceptable that the expression of the arylsulfatase proteion is restricted so that the ratio of the arylsulfatase activity (unit: U/g) to the lactase activity (unit: NLU/g) will be 0.1% or less, preferably 0.02% or less.

Further, although the lactase preparation according to the present invention has a lactase activity of 4,000 NLU/g or more, it is preferably 4,500 NLU/g or more, and more preferably 5,000 NLU/g or more.

The diploid strain of yeast having a lactase gene in which expression of arylsulfatase protein is restricted may be a mutant that has been obtained by treating a diploid strain of yeast to mutate it or may be another mutant that has been obtained by manipulating a diploid strain of yeast to delete an arylsulfatase gene or a gene to regulate expression of an arylsulfatase protein. A mutant is preferable, of which parent strain is a diploid strain of yeast having a large amount of production of lactase protein.

The diploid strain of yeast is preferably a diploid strain of Kluyveromyces lactis or Kluyveromyces marxianus that is closely related to the Kluyveromyces lactis. Further, a gene-recombinant microorganism, in which a lactase gene of yeast has been transformed and expression of arylsulfatase protein is restricted, is preferably a gene-recombinant microorganism, to which a lactase gene of Kluyveromyces lactis or Kluyveromyces marxianus has been transformed.

The present invention also relates to a method for producing a lactase preparation characterized by culturing a diploid strain of yeast having a lactase gene, in which expression of arylsulfatase protein is restricted, or a gene-recombinant microorganism in which a lactase gene of yeast has been transformed and expression of arylsulfatase protein is restricted; gathering yeast or microorganic cells without destroying their cell walls, gathering culture fluid with yeast or microorganic cells after destruction of their cell walls, or gathering culture fluid without destroying cell walls; and preparing a lactase preparation having a lactase activity of 4,000 NLU/g or more according to the FCC IV method and an arylsulfatase activity of 0.1% or less of the lactase activity as the basis, in which the arylsulfatase activity (unit: U/g) has been determined and calculated by the method for determining activity of arylsulfatase in an aqueous system according to the present invention comprising above steps (1) to (10), by using, as a raw material, the gathered yeast or microorganic cells and/or gathered culture fluid without a step for removing arylsulfatase. The case where culture fluid is gathered without destroying cell walls is such a case that the gene-recombinant microorganism secretes lactase.

The steps for preparing lactase preparation can comprise purification steps that are performed in this technical field, such as concentration of lactase protein. However, in the method for producing a lactase preparation according to the present invention, a step for removing arylsulfatase is not performed. Because a diploid strain of yeast or a gene-recombinant microorganism is used, in which it produces lactase protein having high activity and the production amount of arylsulfatase protein contaminated is very small even if it exists, a lactase preparation having high lactase activity can be obtained even though the concentration rate of the produced lactase protein is not large, and because the concentration rate is not large, the arylsulfatase protein contaminated does not become high activity even if it has been concentrated.

Furthermore, the present invention relates to a dairy product that has been produced by using the lactase preparation according to the present invention.

### EFFECTS OF INVENTION

In the method for determining activity of arylsulfatase in an aqueous system according to the present invention, arylsulfatase of much less amount than that of before can be determined by using its activity as an indicator. Because a high sensitive method for determining activity of arylsulfatase was established according to the present invention, it has become possible to exactly know the amount of arylsulfatase in a lactase preparation by using its activity as an indicator.

Also, it has become possible to provide a lactase preparation having high lactase activity and having a very small amount of arylsulfatase contaminated or no arylsulfatase by using, as a raw material, cultured yeast or microorganic cells and/or culture fluid of those cells, wherein the yeast or microorganic cells are those of a diploid strain of yeast having a lactase gene in which expression of arylsulfatase protein is restricted or those of a gene-recombinant microorganism in which a lactase gene of yeast has been transformed and expression of arylsulfatase protein is restricted. Because the lactase preparation of the present invention has high lactase activity, an effect can be attained, of which effect is that the usage of the preparation can be reduced, and thus another effect can also be attained, of which effect is that amounts of additives such as stabilizers or impurities that are introduced to the objective, to which the preparation is added, can be reduced even if the preparation contains the additives or the impurities.

When a diploid strain of yeast is used, there are such advantages that its property is more difficultly altered than that of a monoploid strain even after continuation of subculture, and that the production amount of a protein per a unit amount of culture fluid is generally larger than that of a monoploid strain.

When the lactase preparation of the present invention is used, such an effect can be attained that the development of unreasonable and undesirable taste and smell can be suppressed in shelf-stable milk at ordinary temperatures (UHT milk) or the like.

According to the method for producing a lactase preparation of the present invention, a lactase preparation having high lactase activity is produced without a step for removing arylsulfatase contaminated. Because this method does not comprise "the step for removing arylsulfatase," its production efficiency is high and there is no reduction of lactase activity in purification steps for removing arylsulfatase.

### BRIEF DESCRIPTION OF DRAWINGS

[Figure 1] It is a graph that shows a result of determination of arylsulfatase activity by a colorimetric method.
[Figure 2] It is a graph that shows a result of determination of arylsulfatase activity by a fluorescence method.
[Figure 3] It is a schematic diagram that shows a method for constructing a vector pdSuC1 for disrupting arylsulfatase gene.
[Figure 4] It is a schematic diagram that shows a method for constructing a vector pdSuCM6 for disrupting arylsulfatase gene.
[Figure 5] It is a schematic diagram that shows a method for introducing two vectors for disrupting arylsulfatase genes.
[Figure 6] It is a photograph that shows results of Southern blottings of a strain comprising two arylsulfatase genes, another strain in which one arylsulfatase gene has been disrupted, and the other strain in which two arylsulfatase genes have been disrupted.

### EMBODIMENTS FOR PERFORMING INVENTION

First, a method for determining activity of arylsulfatase will be explained.

The method that has been conventionally known as a method for determining activity of arylsulfatase is a colorimetric one, in which a compound comprising a chromophore such as p-nitrophenol and a sulfate group that is coupled to the chromophore is used as a substrate. In this method, the amount of the chromophore is determined by absorbance, in which the chromophore has been liberated because the sulfate group has left from the substrate by a reaction of the substrate with arylsulfatase. However, when the amount of the liberated chromophore such as p-nitrophenol is low, the change of the absorbance is small and thus it is difficult to obtain a clear determination value.

As a method for determining activity of arylsulfatase, a fluorescence method has also been known (Method in Enzymology, 11/21), in which a compound comprising a fluorophore and a sulfate group that is coupled to the fluorophore, e.g., 4-methylumbelliferone sulfate, is used as a substrate. It is said that the sensitivity of a fluorescence method is generally at least hundred times that of a colorimetric one.

The present inventors have studied the conditions for determination, under which conditions a sensitivity higher than that of a conventional colorimetric or fluorescence method can be attained in a method for determining activity of arylsulfatase in an aqueous system. They have arrived at a version that the arylsulfatase activity can be determined with a high sensitivity by increasing ionic strength in a reaction system of an enzyme with a substrate. Thus, they have established the method for determining activity of arylsulfatase according to the present invention. It has been absolutely unknown in the past that by increasing ionic strength in an aqueous reaction system when arylsulfatase is reacted with its substrate, the enzyme reaction is remarkably activated and as a result a chromophore or fluorophore is liberated in a more amount. By performing the method for determining activity of arylsulfatase in an aqueous system according to the present invention in a fluorescence method, it has become possible to exactly know the amount of arylsulfatase contaminated in a lactase preparation. As a result, it has become possible to provide a lactase preparation, of which arylsulfatase amount is zero or a very, very small.

The method for determining activity of arylsulfatase in an aqueous system according to the present invention is characterized in that when arylsulfatase is reacted with its substrate (with the proviso that the substrate liberates fluorophore or chromophore by suffering an action of the arylsulfatase), ionic strength of the reaction system is increased. Specific means for increasing the ionic strength in the reaction system include one wherein an inorganic salt is coexisted and another one wherein the enzyme reaction is performed in a buffer system.

Examples of inorganic salts that should be added to the reaction system include potassium chloride, sodium chloride, ammonium sulfate, and the like. The concentration of such an inorganic salt is, for example, 10 to 1000mM, and preferably 50 to 500mM in the reaction system. Examples of buffer include phosphate buffers such as phosphoric acid-potassium phosphate buffers (wherein the concept of potassium phosphate includes potassium dihydroxyphosphate, dipotassium hydroxyphosphate, and tripotassium phosphate), phosphoric acid-sodium phosphate buffers (wherein the concept of sodium phosphate includes sodium dihydroxyphosphate, disodium hydroxyphosphate, and trisodium phosphate), and phosphate buffered saline, which do not denature an enzyme protein. The concentration of the buffer in the reaction system is, for example, 10 to 200mM, and preferably 50 to 200mM. To coexist the inorganic salt in the reaction system, for example, an inorganic salt may be added to an aqueous solution of a specimen in which the existence of arylsulfatase is predicted (for example, one obtained by dissolving the specimen in water or a buffer), or an inorganic salt may be added to an aqueous solution of a substrate.

A typical example of the method for determining activity of arylsulfatase in an aqueous system according to the present invention is as follows:

(1) A specimen in which the existence of arylsulfatase is predicted is arbitrarily diluted with 100mM potassium phosphate buffer (pH6.5) comprising 0.5M potassium chloride to obtain a sample.
(2) An aqueous solution comprising potassium 4-methylumbelliferone sulfate in a concentration of 2mM is prepared.
(3) The sample and the aqueous potassium 4-methylumbelliferone sulfate solution are mixed with each other at a ratio of 1:1 (volume basis) and are reacted at 37degrees Celsius for 3 hours.
(4) To the reacted solution, 0.1N aqueous sodium hydroxide solution having the same amount (volume basis) as that of the reacted solution is added to stop the reaction, thus obtaining a sample for determination.
(5) Fluorescence intensity is determined at an excitation wavelength of 360nm and a fluorescence wavelength of 450nm.
(6) 4-Methylumbelliferone is dissolved in 100mM potassium phosphate buffer (pH6.5) comprising 0.5M potassium chloride to obtain a solution having an appropriate concentration, 0.1N aqueous sodium hydroxide solution is added in a similar way as that in step (4), and fluorescence intensity is determined under the same conditions as those in step (5).
(7) From step (6), a calibration curve is prepared.
(8) From the fluorescence intensity that was determined in step (5) and the calibration curve that was prepared in step (7), the concentration of 4-methylumbelliferone of the sample for determination is calculated, and the calculated value is divided by 3, thus obtaining the concentration of the 4-methylumbelliferone in the case where the reaction time of period is 1 hour. Further, from the volume of the reacted solution, the amount of the 4-methylumbelliferone that was liberated by the reaction of 1 hour is calculated.
(9) Because the amount of the 4-methylumbelliferone thus calculated is based on the amount of the specimen that was contained in the sample prepared in step (1), the calculated amount is converted to that of the 4-methylumbelliferone per 1g of the specimen.
(10) When the amount of the 4-methylumbelliferone that was liberated per 1 hour of the time of period of the reaction of the substrate and the enzyme is 1nmole, it is defined as 1 unit(U), and the unit is shown as a unit amount per 1g of the specimen (i.e., an enzyme preparation), namely, "unit(U)/g".

The lactase preparation according to the present invention has a lactase activity of 4,000 NLU/g or more (preferably 4,500 NLU/g or more, still more preferably 5,000 NLU/g or more) according to the FCC IV method (Food Chemicals Codex Fourth Edition, effective July 1, 1996, Committee on Food Chemicals odex p.p.801-802), and an arylsulfatase activity of 0.1% or less (preferably 0.02% or less) of the lactase activity (unit: NLU/g) of the FCC IV method as the basis, in which the arylsulfatase activity is determined and calculated by the method that is described above as a specific example of the method for determining activity of arylsulfatase according to the present invention.

In the production of the lactase preparation according to the present invention, a diploid strain of yeast having a lactase gene is used, in which strain expression of arylsulfatase protein is restricted and by which strain lactase protein is produced. Further, the diploid strain of yeast that is used in the present invention is one that produces lactase protein with a high activity, which can provide a lactase preparation of 4,000 NLU/g or more as it is or by concentrating it. The diploid strain of yeast is, for example, a mutant that can be obtained by treating a microorganism to mutate it. Such a mutant can be obtained by, for example, a method wherein a diploid strain of yeast that produces lactase protein with high activity is exposed to ultraviolet irradiation or a chemical mutagen to perform mutation, thus disrupting or deleting arylsulfatase genes or genes to regulate expression of arylsulfatase protein about both genes of the diploid, or a method wherein arylsulfatase genes or genes to regulate expression of arylsulfatase protein are deleted by genetic engineering procedures about both genes of the diploid. To know whether a desired mutant can be obtained, arylsulfatase activity of a culture fluid in which the mutated yeast has been cultured should be determined by the method (fluorescence method) for determining activity of arylsulfatase according to the present invention

Mutation induction by ultraviolet is performed by, for example, irradiating ultraviolet to a suspension of a diploid yeast. Chemical mutagenesis is performed by, for example, adding a chemical mutagen to a suspension of a diploid yeast. Examples of the chemical mutagen include 5-bromouracil, 2-aminopurine, nitrous acid, hydroxylamine, acriflavine, methanesulfonate compounds, nitrosoguanidine, and the like.

To delete arylsulfatase genes or genes to regulate expression of an arylsulfatase protein by genetic engineering procedures, common genetic engineering procedures should be applied, for example, obtaining a gene fragment having a sequence that is homologous to the sequence of the gene that is intended to be deleted, sub-cloning the fragment to a vector to construct a new vector for disrupting the gene that is intended to be deleted, and transforming a diploid strain of yeast by using the new vector.

In the production of the lactase preparation according to the present invention, a gene-recombinant microorganism that produces lactase protein with high activity can also be used, in which a lactase gene of yeast has been transformed so that lactase protein is expressed and expression of arylsulfatase protein is restricted. As described before, "expression of arylsulfatase protein is restricted" means that arylsulfatase protein is not produced or its production amount is reduced, because, for example, genes that relate to the production of arylsulfatase protein are restricted, specifically because there are no arylsulfatase gene and/or no gene to regulate expression of arylsulfatase protein, or, because an arylsulfatase gene (structure gene) has been disrupted or an expression regulating gene that encourages arylsulfatage gene to express arylsulfatase protein has been disrupted.

The gene-recombinant microorganism in which a lactase gene of yeast has been transformed and expression of arylsulfatase protein is restricted can be produced by a known method. For example, to a plasmid that is tolerant to medicine A, a lactase gene is inserted, with a gene to regulate expression of lactase gene if necessary By using the plasmid to express lactase thus prepared, a microorganism as a host is transformed. The microorganism that has been transformed is cultured in a medium comprising medicine A, and appeared colonies are selected.

As the host to obtain the gene-recombinant microorganism in which lactase gene of yeast has been transformed and expression of arylsulfatase protein is restricted, Escherichia coli, yeast, Bacillus subtilis, and the like can be used.

As the host to obtain the gene-recombinant microorganism in which lactase gene of yeast has been transformed and expression of arylsulfatase protein is restricted, it is preferable to use a host intrinsically having no arylsulfatase gene nor a gene to regulate expression of arylsulfatase protein, or another host of which arylsulfatase gene or a gene to regulate expression of arylsulfatase protein has been disrupted or deleted.

The lactase for the lactase preparation is produced by culturing a diploid strain of yeast having a lactase gene in which expression of arylsulfatase protein is restricted or a gene-recombinant microorganism in which a lactase gene of yeast has been transformed and expression of arylsulfatase protein is restricted, wherein the diploid strain of yeast and the microorganism produce lactase protein having high activity; gathering the yeast or microorganic cells without destroying their cell walls, gathering culture fluid with yeast or microorganic cells after destruction of their cell walls, or gathering culture fluid without destroying cell walls; and using as a raw material the gathered yeast or microorganic cells and/or the culture fluid, without any step for removing arylsulfatase. The concept of "culture fluid" includes also a culture supernatant.

To culture microorganism such as yeast, an incubator such as a flask, a jar, and a tank can be used. As the culturing conditions, temperature, pH, a stirring number, and the like, which are suitable for an enzyme production by the microoraganism, are selected.

After the completion of culture, a culture fluid comprising lactase dissolved is obtained by generally destroying cell walls. In the case where the microorganism that has been cultured secretes lactase, it may be unnecessary to destroy the cell walls. In the case where a culture fluid is not used and only yeast cells (or microorganic cells) have been gathered, then cell walls of the yeast cells (or microorganic cells) are destroyed in distilled water, and constituents contained in the cells are dissolved in the distilled water to be an aqueous solution comprising the yeast cells (or microorganic cells).

The culture fluid or the aqueous solution comprising or not comprising the yeast or microorganic cells is generally divided into supernatant and residues by an appropriate method such as centrifugation, filtration, or the like, which is commonly performed in this technical field. The supernatant may be used as an enzyme fluid as it is. Or after its concentration is increased by using ultrafiltration membrane or the like, the concentrated one may be used as an enzyme fluid. The enzyme fluid may be pulverized by a method such as spray dry, freeze dry, or the like. The enzyme fluid itself can also be used as a lactase preparation.

The lactase preparation according to the present invention is produced by using a diploid strain of yeast or a microorganism that produces lactase protein having high activity and does not produce arylsulfatase protein or produces it only infinitesimal quantity. Therefore, generally it is unnecessary to do, for removing arylsulfatase only, one or more operations among purification operations such as adsorption, chromatography, crystallization, and the like by using the culture fluid or the like. The method for producing a lactase preparation according to the present invention does not include any step for removing arylsulfatase. As described before, purification methods by which lactase protein is not separated from arylsulfatase protein, such as solvent fractionation, ammonium sulfate fractionation, and the like, are not included within the definition of "a step for removing arylsulfatase."

An essential constituent of the lactase preparation according to the present invention is lactase. In the lactase preparation, any other constituent may exist, as long as the substance does not inhibit the activity of lactase and its amount is one by which the activity of lactase is not inhibited; or as long as the substance does not undesirably interact with an objective for which the lactase preparation is used. Examples of the substance that may exist include those that contribute stabilization of lactase, such as metallic salts, various sugars, ascorbic acid, glycerol, and the like, excipients that are used to increase usability, such as starches and dextrin, and inorganic salts and the like that have buffering action. The state of the lactase preparation is not particularly restricted. Its state may be, for example, powder, granule, solution, or the like.

The present invention also relates to dairy products that have been produced by using the lactase preparation according to the present invention. The dairy products include milks such as shelf-stable milk at ordinary temperatures (UHT milk), yoghurts, fresh creams, sour creams, cheeses, and the like. The lactase preparation is used by a method and in usage (with the proviso that the amount is calculated based on its lactase activity) that are common in this technical field.

### EXAMPLES

Below, the present invention will be specifically explained by referring to Examples.

### [Example 1] Development of method for determining arylsulfatase activity (Part 1)

GODO-YNL2 (liquid lactase preparation, produced by Godo Shusei Co. Ltd.) was subjected to 5-fold dilution with 100mM potassium phosphate buffer (pH 6.5) containing 0, 0.2, 0.5, or 1.0M potassium chloride. To 0.5mL of each solution, 0.5mL of p-nitrophenyl sulfate in 100mM potassium phosphate buffer (pH 6.5) was added and the solution was allowed to react at 37 degrees Celsius for 3 hours. The reaction was stopped by adding to the solution 1.5mL of 1.5N aqueous sodium hydroxide solution and the absorbance was determined at 410 nm. Relative values are shown in Table 1, in which the case where there is no potassium chloride is specified as 100%.

**[Table 1]**

| Increase of Determined Value of Arylsulfatase Activity by Potassium Chloride | | | | |
|---|---|---|---|---|
| Concentration of Potassium Chloride in Reaction System (M) | 0 | 0.1 | 0.25 | 0.5 |
| Relative Value (%) | 100 | 230 | 350 | 420 |

As shown in Table 1, the determined values of arylsulfatase activity increased by the addition of potassium chloride. In other words, by the addition of potassium chloride, a higher sensitive assay was able to be attained.

### [Example 2] Development of method for determining arylsulfatase activity (Part 2)

(1) GODO-YNL2 (liquid lactase preparation, produced by Godo Shusei Co. Ltd.) was subjected to 100-fold dilution with 100mM potassium phosphate buffer (pH 6.5) containing 0, 125, 250, 500, or 1,000mM sodium chloride. To 0.5mL of each solution, 0.5mL of 2mM aqueous potassium 4-methylumbelliferone sulfate solution was added and the solution was allowed to react at 37 degrees Celsius for 3 hours. The reaction was stopped by adding to the solution 1.0mL of 0.1N aqueous sodium hydroxide solution and the fluorescence intensity was determined at an excitation wavelength of 360nm and a fluorescence wavelength of 450nm. Relative values are shown in Table 2, in which the case where there is no sodium chloride is specified as 100%.
(2) The reaction and determination of fluorescence were performed in the same way as those described in the above (1), except that 100mM sodium phosphate buffer (pH 6.5) was used as the diluent for the enzyme instead of 100mM potassium phosphate buffer (pH 6.5). The results are shown in Table 2.
(3) The reaction and determination of fluorescence were performed in the same way as those described in the above (1), except that potassium chloride was added to the diluent for the enzyme instead of sodium chloride. The results are shown in Table 2.
(4) The reaction and determination of fluorescence were performed in the same way as those described in the above (1), except that 100mM sodium phosphate buffer (pH 6.5) was used instead of 100mM potassium phosphate buffer (pH6.5) as the diluent for the enzyme, and that potassium chloride was added to the diluent for the enzyme instead of sodium chloride. The results are shown in Table 2.

**[Table 2]**

| Increase of Determined Value of Arylsulfatase Activity by Combination of Inorganic Salt and Buffer | | | | | | |
|---|---|---|---|---|---|---|
| Type of Buffer and Concentration in Reaction System | Concentration of Inorganic Salt in Reaction System (mM) | Relative Value (%) | | | | |
| | | 0 | 62.5 | 125 | 250 | 500 |
| | Type of Inorganic Salt | | | | | |
| 50mM Potassium Phosphate Buffer | Sodium Chloride | 100 | 230 | 310 | 440 | 600 |
| 50mM Sodium Phosphate Buffer | Sodium Chloride | 100 | 200 | 310 | 410 | 550 |
| 50mM Potassium Phosphate Buffer | Potassium Chloride | 100 | 230 | 330 | 460 | 640 |
| 50mM Sodium Phosphate Buffer | Potassium Chloride | 100 | 220 | 340 | 470 | 600 |

As shown in Table 2, the determined values of arylsulfatase activity increased at an approximately similar rate when sodium chloride or potassium chloride was added as an inorganic salt, and the reaction and determination were performed in potassium phosphate or sodium phosphate buffer.

### [Example 3] Development of method for determining arylsulfatase activity (Part 3)

(1) GODO-YNL2 (liquid lactase preparation, produced by Godo Shusei Co. Ltd.) was subjected to 100-fold dilution with 100mM, 125mM, 250mM, 500mM, or 1,000mM potassium phosphate buffer (pH 6.5). To 0.5mL of each solution, 0.5mL of 2mM aqueous potassium 4-methylumbelliferone sulfate solution was added and the solution was allowed to react at 37 degrees Celsius for 3 hours. To the solution, 1.0mL of 0.1N aqueous sodium hydroxide solution (for 100mM, 125mM, and 250mM potassium phosphate buffers) or 1.0N aqueous sodium hydrochloride solution (for 500mM and 1,000mM potassium phosphate buffers) was added to stop the reaction, and the fluorescence intensity was determined at an excitation wavelength of 360nm and a fluorescence wavelength of 450nm. Relative values are shown in Table 3, in which the case where 100mM potassium phosphate buffer was used is specified as 100%.
(2) The reaction was performed in the same way as that described in the above (1), except that the enzyme was diluted with 100mM potassium phosphate buffer (pH 6.5) and 0, 125, 250, 500, or 1,000mM ammonium sulfate was added. For the system to which 1,000mM ammonium sulfate had been added, 1.0mL of 1.0N aqueous sodium hydroxide solution was added to stop the reaction, and for other systems, 1.0mL of 0.1N aqueous sodium hydroxide solution was added to stop the reaction. The fluorescence intensity was determined at an excitation wavelength of 360nm and a fluorescence wavelength of 450nm. Relative values are shown in Table 4, in which the determined value of activity of the system comprising no ammonium sulfate added is specified as 100%.
(3) The reaction and determination of fluorescence were performed in the same way as those described in the above (2), except that 0, 125, 250, 500, or 1,000mM glucose was added to the diluent of the enzyme instead of ammonium sulfate. The results are shown in Table 4.

**[Table 3]**

| Increase of Determined Value of Arylsulfatase Activity by Potassium Phosphate Buffer | | | | | |
|---|---|---|---|---|---|
| Concentration of Potassium Phosphate Buffer in Reaction System (mM) | 50 | 62.5 | 125 | 250 | 500 |
| Relative Value (%) | 100 | 140 | 190 | 160 | 170 |

**[Table 4]**

| Increase of Determined Value of Arylsulfatase Activity by Combination of Additive and Buffer | | | | | | |
|---|---|---|---|---|---|---|
| Type of Buffer and Its Concentration in Reaction System | Concentration of Additive in Reaction System (mM) | Relative Value (%) | | | | |
| | | 0 | 62.5 | 125 | 250 | 500 |
| | Type of Additive | | | | | |
| 50mM Potassium Phosphate Buffer | Ammonium Sulfate | 100 | 270 | 370 | 390 | 400 |
| 50mM Potassium Phosphate Buffer | Glucose | 100 | 140 | 190 | 160 | 170 |

As shown in Table 3, although the increase in buffer concentration also contributed to rise of determined values of arylsulfatase activity, the effect was small. Further, as shown in Table 4, the addition of ammonium sulfate increased the determined values of arylsulfatase activity, whereas glucose showed a minor effect to increase the determined values of arylsulfatase activity.

### [Example 4] Confirmation of step in which effect of addition of inorganic salt is arisen.

It was confirmed that the effect of addition of an inorganic salt is due to promoted enzyme reaction or enhanced fluorescence intensity of fluorophore.

GODO-YNL2 (liquid lactase preparation, produced by Godo Shusei Co. Ltd.) was subjected to 100-fold dilution with 100mM potassium phosphate buffer (pH 6.5). Further, it was separately subjected to 100-fold dilution with 100mM potassium phosphate buffer (pH 6.5) containing 0.5M potassium chloride. To 0.5mL of each solution, 0.5mL of 2mM aqueous potassium 4-metylumbeliferone sulfate solution was added and the solution was allowed to react at 37 degrees Celsius for 1 hour. To the solution, 1.0 mL of 0.1N aqueous sodium hydroxide solution was added to stop the reaction. To 200µL of each solution after stopping the reaction, 200µL of an aqueous solution containing potassium chloride at a concentration of 0mM, 125mM, 250mM, 500mM, or 1,000mM was added, and the fluorescence intensity was determined at an excitation wavelength of 360nm and a fluorescence wavelength of 450nm. As a blank, a solution was used, which solution had been prepared by adding 0.1N aqueous sodium hydroxide solution to a diluted enzyme solution to inactivate and then adding aqueous potassium 4-metylumbelliferon sulfate solution.

Relative values are shown in Table 5, in which the case where the lactase preparation that had been subjected to 100-fold dilution with 100mM potassium phosphate buffer (pH 6.5) was used for the reaction and potassium chloride was not added after stopping the reaction (200µL of distilled water with potassium chloride concentration of 0mM was added) is specified as 100%.

**[Table 5]**

| Confirmation of Step in which Effect by Addition of Inorganic Salt Is Shown | | | | | |
|---|---|---|---|---|---|
| | Relative Value (%) | | | | |
| Concentration of Potassium Chloride (mM) in Aqueous Potassium Chloride Solution That Was Added after Stopping Reaction | 0 | 125 | 250 | 500 | 1000 |
| Condition during Reaction | | | | | |
| In Case where Reaction Was Performed in 50mM Potassium Phosphate Buffer (%) | 100 (basis) | 100 | 100 | 100 | 100 |
| In Case where Reaction Was Performed in 50mM Potassium Phosphate Buffer + 250mM Potassium Chloride (%) | 520 | 520 | 520 | 510 | 490 |

As shown in Table 5, when the inorganic salt had been added before starting the reaction, i.e., when the enzyme reaction was performed in the presence of the inorganic salt, the fluorescence intensity was enhanced, whereas the fluorescence intensity was not enhanced, when the inorganic salt was added after the completion of the enzyme reaction. Accordingly, it became clear that because the inorganic salt promoted the enzyme reaction to increase the absolute amount of the fluorophore liberated, the fluorescence intensity was enhanced.

### [Example 5] Determination of arylsulfatase activity by conventional colorimetric method

GODO-YNL2 (liquid lactase preparation, produced by Godo Shusei Co. Ltd.) was diluted with distilled water to obtain a 1%(w/v) solution. The solution was diluted with 100mM potassium phosphate buffer (pH 6.5) containing 0.5M potassium chloride to obtain 0.8%(w/v), 0.6%(w/v), 0.4%(w/v), and 0.2%(w/v) solutions. To 0.5mL of each solution, 0.5mL of 100mM potassium phosphate buffer (pH 6.5) containing 20mM p-nitrophenyl sulfate was added and the solution was allowed to react at 37 degrees Ceisius for 3 hours. The reaction was stopped by adding to this solution 1.5mL of 1.5N aqueous sodium hydroxide solution, and the absorbance was determined at 410nm.

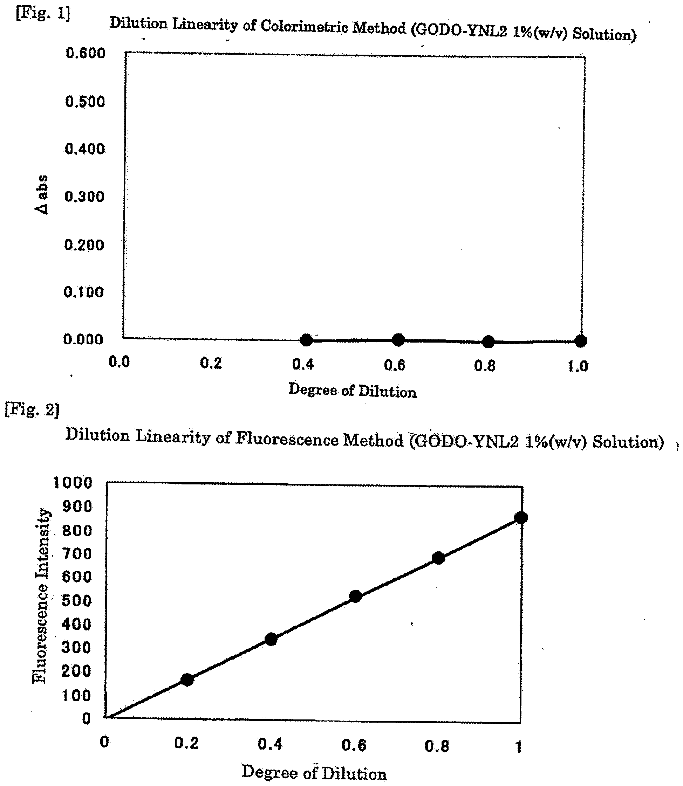

The results are shown in Figure 1. By the colorimetric method, no arylsulfatase activity was detected for the 1%(w/v) solution of the enzyme preparation.

### [Example 6] Determination of arylsulfatase activity by fluorescence method

GODO-YNL2 (liquid lactase preparation, produced by Godo Shusei Co. Ltd.) was diluted with 100mM potassium phosphate buffer (pH 6.5) containing 0.5M potassium chloride to obtain a 1%(w/v) solution. This 1% solution was further diluted with the same buffer to obtain 0.8%(w/v), 0.6%(w/v), 0.4%(w/v), and 0.2%(w/v) solutions.

To 0.5mL of each solution, 0.5mL of 2mM aqueous potassium 4-methylumbelliferyl sulfate solution was added and the solution was allowed to react at 37 degrees Celsius for 3 hours. To this solution, 1.0mL of 0.1N aqueous sodium hydroxide solution was added to stop the reaction, and the fluorescence intensity was determined at an excitation wavelength of 360nm and a fluorescence wavelength of 450nm.

The results are shown in Figure 2. By the fluorescence method, unlike the colorimetric method, the solutions of the enzyme preparation having its concentration of 1%(w/v) or lower also showed good quantitative performance.

### [Example 7] Comparison of colorimetric and fluorescence methods in arylsulfatase activity determination

The difference between sensitivities of colorimetric and fluorescence methods in the determination of arylsulfatase activity was studied. First, purified lactase for use in this experiment was prepared.

### (Preparation of purified lactase)

To 50kg of GODO-YNL2 (liquid lactase preparation, produced by Godo Shusei Co. Ltd.), water was added to desalt by using an ultrafiltration membrane (ACP membrane, produced by Asahi Kasei Corp.) until the conductivity became 3mSv or lower. The total amount was adjusted to 125L by adding water. Then, it was adsorbed to ion exchange resin (DEAE TOYOPEARL 650M, 40cmϕ, 50L, produced by Tosoh Corp.) pre-equilibrated with 10mM potassium phosphate buffer (pH 7). The resin was washed with 40L of 10mM potassium phosphate buffer (pH 7) containing 50mM sodium chloride, and then lactase was eluted with 200L of 10mM potassium phosphate buffer (pH 7) containing 100mM sodium chloride. Upon elution, the eluate was divided into 20L fractions. The lactase activity (by the FCC IV method; Food Chemicals Codex 4th Edition, Effective July 1st, 1996, Committee on Food Chemicals Codex, p.p.801-802) and arylsulfatase activity (by the fluorescence method; it will be described below in detail) of each fraction were determined, and fractions with reduced arylsulfatase were collected, mixed, and concentrated using an ultrafiltration membrane (ACP membrane, produced by Asahi Kasei Corp.) to obtain a concentrated lactase solution. To this concentrated solution, glycerol was added to be 50%(w/v). Thus, a purified lactase preparation was obtained.

Arylsulfatase activity of GODO-YNL2 (liquid lactase preparation, produced by Godo Shusei Co. Ltd.) and the purified lactase preparation prepared as described above, both of which had lactase activity by the FCC IV method of 5,000 to 5,500 NLU/g, were determined (the fluorescence method; it will be described below in detail). The arylsulfatase activity of purified lactase preparation was determined as being 1/840 as compared to that of pre-purification (see Table 6 below).

### (Preparation of enzyme preparations with various contamination rates of arylsulfatase)

The purified lactase preparation prepared as described above and GODO-YNL2 (liquid lactase preparation, produced by Godo Shusei Co. Ltd.) were appropriately mixed to prepare lactase preparations with various contamination rates of arylsulfatase, and the lactase activity (by the FCC IV method) and the arylsulfatase activity (by the colorimetric and fluorescence methods) were determined.

### (Determination of arylsulfatase activity by colorimetric method)

To 0.5mL of the lactase preparation, 0.5mL of 100mM potassium phosphate buffer (pH 6.5) containing 20mM p-nitrophenyl sulfate was added and the solution was allowed to react at 37 degrees Celsius for 3 hours. To this solution, 1.5mL of 1.5N aqueous sodium hydroxide solution was added to stop the reaction, and the absorbance was determined at 410 nm.

Separately, aqueous solutions containing p-nitrophenol at a concentration of 0 to 0.5mM were prepared. To 0.5mL of each solution, 0.5mL of 100mM potassium phosphate buffer (pH 6.5) was added. Further, 1.5mL of 1.5N aqueous sodium hydroxide solution was added to obtain samples for determination. The absorbance at 410nm was determined to prepare a calibration curve.

From the calibration curve, the concentration of p-nitrophenol contained in 1mL of the reaction solution was determined, and divided by 3 (because the reaction time was 3 hours), to calculate the p-nitrophenol concentration for the reaction time of period of 1 hour. Then, from this concentration, the amount of p-nitrophenol contained in 1mL of the reaction solution (unit: nmole) was calculated, and multiplied by 2 (for converting the value into per 1g, because the amount of lactase preparation used was 0.5g), to calculate the arylsulfatase activity. One U corresponds to the activity which produces 1nmole of p-nitrophenol in 1 hour, and the arylsulfatase activity is represented by the unit "U/g-enzyme preparation."

### (Determination of arylsulfatase activity by fluorescence method)

The lactase preparation was diluted with 100mM potassium phosphate buffer (pH 6.5) containing 0.5M potassium chloride to obtain a 1%(w/v) solution. To 0.5mL of this 1% solution, 0.5mL of 2mM aqueous potassium 4-methylunbelliferyl sulfate solution was added and the solution was allowed to react at 37 degrees Celsius for 3 hours. To the solution 1mL of 0.1N aqueous sodium hydroxide solution was added to stop the reaction, and the fluorescence intensity was determined at an excitation wavelength of 360nm and a fluorescence wavelength of 450nm.

Separately, 100mM potassium phosphate buffers (pH 6.5) containing 0.5M potassium chloride and 4-methylumbelliferone at a concentration of 0 to 4µM were prepared. To 1.0 mL of each solution, 1mL of 0.1N aqueous sodium hydroxide solution was added to obtain samples for determination. The fluorescence intensity was determined at an excitation wavelength of 360nm and a fluorescence wavelength of 450nm to prepare a calibration curve.

From the calibration curve, the concentration of 4-methylumbelliferone contained in 1mL of the reaction solution was determined and it was divided by 3 (because the reaction time of period was 3 hours). Then, from the volume of the reaction solution, the absolute amount of 4-methylumbelliferone produced during the reaction was calculated. The amount was further multiplied by 200 (for converting the value into per 1g of the specimen (lactase preparation), because the amount of lactase preparation used was 0.5×0.01=0.005g), and thereby arylsulfatase activity was calculated. One U is such an activity that 1nmole of 4-methylumbelliferone is produced in 1 hour, and the arylsulfatase activity is represented by the unit "U/g-enzyme preparation."

### (Results)

The results are shown in Table 6. By the colorimetric method, determination was not possible when the content of arylsulfatase decreased, whereas by the fluorescence method it was possible to accurately determine until the concentration area of about 1/100 of the detection limit by the colorimetric method.

**[Table 6]**

| Lactase Activity and Arylsulfatase Activity of Lactase Preparations Containing Arylsulfatase in Various Contamination Rates | | | | |
|---|---|---|---|---|
| | Lactase Activity (NLU/g) | Arylsulfatase Activity (U/g) | | Arylsulfatase Activity of This Invention / Lactase Activity (%) |
| | | Conventional Colorimetric Method | Method of This Invention | |
| GODO-YNL2 | 5400 | 39 | 84 | 1.56 |
| Mixture of GODO-YNL2 with Purified Lactase | (5500) | 8.7 | 20 | 0.36 |
| | (5400) | ND | 15 | 0.28 |
| | (5300) | ND | 1 | 0.02 |
| Purified Lactase | 5500 | ND | 0.1 | 0.002 |

| | | | | |
|---|---|---|---|---|
| ND: Not Detected | | | | |

### [Example 8] Comparison of fluorescence method and the method as stated in WO07/060247 (Japanese Patent Laid-open No. 2009-517061) in determination of arylsulfatase activity, and organoleptic examination for taste (Part 1)

In WO07/060247, it is described that a lactase preparation contaminated with 19 units (units that are defined in WO07/060247 or less of arylsulfatase does not produce off-flavor when the preparation is added to decompose lactose after sterilization of milk. However, this is an observation based on an examination that was performed with the limited lactase reaction time of period of 2 days. On the other hand, when a lactase preparation is actually added to shelf-stable milk at ordinary temperatures (UHT milk), it is thought that enzyme reaction would proceed for a long term of 1 month or more. Therefore, lactase preparations containing arylsulfatase at various contaminating rates were prepared and their taste was confirmed after predetermined days from the addition of the preparations to milk.

### (Preparation of lactase preparations with various contamination rates of arylsulfatase)

Lactase preparations A to E having various contamination rates of arylsulfatase were produced by appropriately mixing the purified lactase preparation prepared in Example 7 and selected GODO-YNL2 (liquid lactase preparation, produced by Godo Shusei Co. Ltd.) containing 100units (based on that described in WO07/060247) of arylsulfatase activity.

The arylsulfatase activity of each of thus prepared lactase preparations A to E was determined by the method described in WO07/060247 and the fluorescence method shown in Example 7 above. Further, the lactase activity was determined by the FCC IV method. The results are shown in Table 7. In this Table, the unit as described in WO07/060247 means ΔOD₄₁₀×10⁶/hour/NLU.

**[Table 7] Arylsulfatase Activity in Lactase Preparations Containing Arylsulfatase in Various Contamination Rates**

| Name of Lactase Preparation and Lactase Activity (NLU/g) | Arylsulfatase Activity (unit) that Was Determined by the Method Described in WO07/060247 (Japanese Patent Laid-open No. 2009-517061) | Arylsulfatase Activity (U/g) that Was Determined by the Method of Present Invention | Arylsulfatase Activity of This Invention / Lactase Activity (%) |
|---|---|---|---|
| A (5500) | ND | 0.1 | 0.002 |
| B (5500) | ND | 1.0 | 0.02 |
| C (5500) | 8 | 27 | 0.5 |
| D (5500) | 18 | 59 | 1.1 |
| E (5500) | 100 | 320 | 5.8 |

| | | | |
|---|---|---|---|
| ND: Not Detected | | | |

### (Organoleptic examination of taste)

Referring to Example 4 of WO07/060247, each of the lactase preparations A to E was added to a commercially available cow milk (heat-sterilized one; sterile conditions: 130 degrees Celsius, 2 seconds) so that the lactase content would become 20,000 NLU/L-milk, and was kept at 30 degrees Celsius. After 2 days, 1 month, and 3 months of storage, an organoleptic examination was performed for the cow milk to which no lactase preparation had been added and the milks to which lactase preparations had been added.

The organoleptic examination was performed as a blind study. Eleven to thirteen panelists smelled and held in mouth the milk after storage for a certain period of time to judge the presence or absence of off-flavor. The evaluation was performed by scoring 0 point (-) for no off-flavor, 1 point (+) which meant that the panelist was aware of off-flavor, or 2 points (++) which meant that the panelist was strongly aware of off-flavor. The results are summarized in Table 8.

**[Table 8]**

| Results of Organoleptic Examination of Taste (Off-flavor) of Cow Milks that Were Treated by Lactase Preparations Containing Arylsulfatase in Various Contamination Rates | | | |
|---|---|---|---|
| Lactase Preparations | Reaction Time of Period | | |
| | 2 days | 1 month | 3 months |
| Not Added | - | - | - |
| A | - | - | - |
| B | - | - | - |
| C | - | ++ | ++ |
| D | - | ++ | ++ |
| E | + | ++ | ++ |

| | | | |
|---|---|---|---|
| ++: Strongly Aware +: Aware -: Not Aware | | | |

After 2 days of the reaction time of period by the lactase preparation (i.e., storage time of period of cow milk), only preparation E exhibited apparent off-flavor. This was coincided with the description in WO07/060247. However, when the reaction time of period was 1 month or longer, preparations C and D also exhibited noticeable off-flavor. This indicates that unusual taste and off-flavor cannot be sufficiently controlled even though the arylsulfatase activity is 8 units, which is the detection limit in the unit described in WO07/060247, considering that the lactase preparations is used in, e.g., shelf-stable milk at ordinary temperatures (UHT milk).

On the other hand, preparations A and B exhibited no noticeable off-flavor for the reaction time of period of 1 and 3 months. Considering the use of the lactase preparation in shelf-stable milk at ordinary temperatures (UHT milk), it is necessary to assume a reaction time of period of 1 month or longer. Therefore, for such usage, it became clear that it is preferable to use lactase preparation A or B (i.e., one having a ratio of the arylsulfatase activity by the method of present invention to the lactase activity by the FCC IV method of 0.02% or less) and that it is more preferable to use lactase preparation A. Further, it became clear that it is necessary to determine the arylsulfatase activity value which is comparable to that of lactase preparation A or B by the fluorescence method as described in this description or by an analysis method having a sensitivity similar to or higher than the above fluorescence method.

### [Example 9] Obtaining a mutant having a reduced arylsulfatase producibility

A loopful of Kluyveromyces lactis G14-427, which was a diploid strain, was inoculated into 10ml of YPD medium (containing 1% of yeast extract, 1% of glucose, and 2% of peptone). The obtained suspension of cells was stored at 30 degrees Celsius to cultivate the cells. After arriving at a logarithmic phase, the suspension was centrifuged and then the cells were gathered. The gathered cells were dispersed in sterile water so that the absorbance of the obtained suspension would become 0.5 at 600nm. By using a UV lamp, ultraviolet was irradiated to the suspension for 15 seconds. The cells were gathered by centrifugation, and then the gathered cells were dispersed in YPD medium by mixing. From the YPD medium comprising the cells, an optimal dose of the YPD medium was taken and applied onto a YPD agar plate. Static culture of the plate was performed at 37 degrees Celsius for 7 days. From a colony that had grown a small amount of cells were scratched, and then the scratched cells were mixed with 1ml of a solution comprising Zymolyase (produced by Seikagaku Bio-business Corporation) in an amount of 1 mg/mL. Reaction was performed at 30 degrees Celsius for 2 hours to destroy cell walls. Thereafter centrifugation was performed and supernatant was gathered.

Lactase activity (the FCC IV method) and arylsulfatase activity (by the fluorescence method disclosed in Example 7) of the supernatant were determined. The ratio of the arylsulfatase activity to the lactase activity was calculated and strains that exhibit small values were selected.

Selected strains were repeatedly subjected to the above treatments for mutation and selection. As a result, a mutant (SM1182 strain) was able to be obtained, in which strain one arylsulfatase gene among two arylsulfatase genes that had existed in the diploid strain, Kluyveromyces lactis G14-427, became dysfunctional. The judgment that "one arylsulfatase gene among two arylsulfatase genes became dysfunctional" was based on the following fact: the culture supernatant of SM1182 strain exhibited an arylsulfatase activity of about one-half of that of the culture supernatant of the mother strain, G14-427 strain.

Furthermore, the obtained mutant (SM1182 strain) was used as a mother strain and treatments to cause mutation were performed. As a result, a mutant (SF-81 strain) in which the other arylsulfatase gene had also become dysfunctional, namely, one having an arylsulfatase activity of zero, was obtained,.

Kluyveromyces lactis G14-427 as a mother strain and two mutants that had been obtained by the methods described above were respectively cultivated by shaking in YPD medium (70 mL/flask) at 26 degrees Celsius for 4 days.

Thereafter, to each culture medium Zymolyase (produced by Seikagaku Bio-business Corporation) was added to be a concentration of 2 mg/mL. Reaction was performed at 30 degrees Celsius for 2 hours to destroy cell walls. Supernatants were respectively gathered by centrifugation, and were subjected to determinations of the lactase activity by the FCC IV method and the arylsulfatase activity by the method disclosed in Example 7. Table 9 shows the result.

**[Table 9]**

| Comparison of Arylsulfatase Activity of Parent Strain and Mutant Strain | | |
|---|---|---|
| | Lactase Activity (NLU/g) | Arylsulfatase Activity (U/g) |
| G14-427 Strain | 16 | 6 |
| SM1182 Strain | 16 | 3 |
| SF-81 Strain | 16 | 0 |

### [Example 10] Obtaining host strain

To 10mL of YPD medium, a loopful of Kluyveromyces lactis G14-427 strain, which was a diploid strain, was inoculated, and the cells were grown to log phase at 30 degrees Celsius. The cells were gathered by centrifuging the culture medium. The gathered cells were dispersed in sterile water so that the absorbance of the obtained suspension would become 0.5 at 600nm. By using a UV lamp, ultraviolet was irradiated to the cell suspension for 15 seconds. The cells were gathered by centrifugation, and mixed and dispersed in YPD medium. An appropriate amount of YPD medium containing the cells was taken and was spread on a YPD agar plate. Static culture of the plate was performed at 37 degrees Celsius for 4 days under static conditions. The colonies that had grown were cultured on SD medium (0.67% amino acid-free yeast nitrogen base, 2% glucose, 2% agar) after their replica-plating, and those that had not be able to grow were selected.

These strains that had not be able to grow on the above SD medium were spread on another SD medium containing 20 mg/L of L-methionine from the original YPD agar plate, and those that had grown were designated as L-methionine-requiring mutant (7-19 strain).

To 10mL of YPD medium, a loopful of the 7-19 strain was inoculated, and the cells were grown to log phase at 30 degrees Celsius. The cells were gathered by centrifuging the culture medium. The gathered cells were dispersed in sterile water so that the absorbance of the obtained suspension would become 0.5 at 600nm. By using a UV lamp, ultraviolet was irradiated to the cell suspension for 15 seconds. The cells were gathered by centrifugation, and mixed and dispersed in YPD medium. An appropriate amount of YPD medium containing the cells was taken and was spread on a YPD agar plate. Static culture of the plate was performed at 37 degrees Celsius for 4 days. The colonies that had grown were cultured on SD medium containing 20 mg/L of L-methionine after their replica-plating, and those that had not be able to grow were selected.

These strains that had not be able to grow on the above SD medium containing L-methionine were spread on another SD medium containing 20 mg/L of L-histidine and 20 mg/L of L-methionine from the original YPD agar plate, and those that had grown were designated as L-methionine and L-histidine double nutrient-requiring mutant (8-23 strain). The growth of nutrient-requiring mutants obtained is shown in Table 10.

**[Table 10]**

| Growth of Mutant Strains in Various Culture Media | | | |
|---|---|---|---|
| | SD Medium | SD Medium + L-Methionine | SD Medium + L-Methionine + L-Histidine |
| G14-427 Strain | + | + | + |
| 7-19 Strain | - | + | + |
| 8-23 Strain | - | - | + |

| | | | |
|---|---|---|---|
| +: Grew -: Not Grew | | | |

### [Example 11] Obtaining gene double-disrupted strain which does not produce arylsulfatase

### (Obtaining host strain)

It was confirmed that in the 8-23 strain that was the L-histidine and L-methionine double nutrient-requiring mutant obtained in Example 10, the nutrient requirements would be complemented by HIS4 gene and MET6 gene, respectively, introduced by the lithium acetate method.

### (Obtaining genomic DNA)

Kluyveromyces lactis G14-427 strain, which was a diploid strain, was cultured in YPD medium. From the culture fluid obtained, genomic DNA was prepared using Dr. GenTLE™ (from yeast) (produced by Takara Bio Inc.). The manipulation was performed according to the instructions in the operating manual attached to Dr. GenTLE™ (from yeast).

### (Construction of vector for disrupting arylsulfatase gene)

Primers SuC-F and SuC-R were designed so that a fragment containing the open reading frame of arylsulfatase gene would be obtained. The sequences of used primers including them were as shown in Table 11.

**[Table 11]**

| Used Primer | |
|---|---|
| Name of Primer | Sequence (5' → 3') |
| SuC-F | ATGACCAAAACAGATGAACCTA |
| SuC-R | CCAGTCTCTTGCGTCGTGA |
| BGLHIS4-F | GAAGATCTCAGACCTGAGGTAACGTTTC |
| HIS4-R | CTGGGTAACTTGTTCTGGTG |
| SuCd-M6F | ATCGATGTTGTTGAGCGGTACTGACAACCAT- -TTGGCAGGTTTCATCAACACACGAGCCG |
| SuCd-M6R | ATCGATAACTCTACCGATATTTTGGTCTAATT- -CATCAACCACTGTTGGTGGAG |

A DNA fragment was obtained by performing PCR under the following conditions with the genomic DNA prepared in advance as the template by using the above primers. As the polymerase, Takara Ex Taq (registered trademark; produced by Takara Bio Inc.) was used and the manipulation was performed according to the attached instructions.

### (PCR conditions)

| | |
|---|---|
| Stage 1 (1 cycle) | 94 degrees Celsius, 3 min. |
| Stage 2 (30 cycles) | 94 degrees Celsius, 1 min. |
| | 54 degrees Celsius, 1 min. |
| | 72 degrees Celsius, 3 min. |
| Stage 3 (1 cycle) | 72 degrees Celsius, 10 min. |
| | Kept at 4 degrees Celsius |

The obtained fragment was purified by MagExtractor™-PCR & Gel Clean up- (produced by Toyobo Co., Ltd.), and then a ligation reaction of the fragment with pGEM (registered trademark) - T vector (produced by Promega) was performed. For the ligation reaction, DNA Ligation Kit <Mighty Mix> (produced by Takara Bio Inc.) was used. The methods of using were as stated in respective attached documents. Then, competent cells of E. coli DH5α strain that had been prepared according to the Hanahan method (Hanahan, D., J. Mol. Biol., 166, 557 (1983)) were transformed, and from the culture fluid of the obtained transformant, plasmid was extracted by using MagExtractor™ - Plasmid - (produced by Toyobo Co., Ltd.). The method of using was as stated in the attached document. As a result, plasmid pGSuC, in which the intended fragment had been subcloned, was obtained.

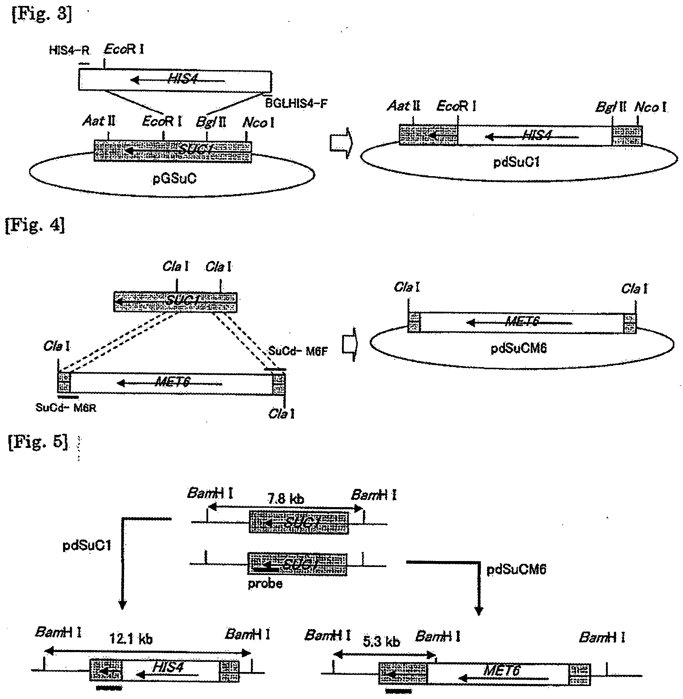

Further, by performing PCR using primers BGLHIS4-F and HIS4-R with genomic DNA as the template, a fragment containing HIS4 gene was obtained. PCR was performed as the method described above. As shown in Figure 3, the obtained fragment containing HIS4 gene was treated with Bgl II and Eco RI and then it was inserted into the Bgl II-EcoRI site of pGSuC. The thus constructed plasmid was designated as pdSuC1. The various methods used for construction were similar to those stated above.

To construct a vector for disrupting arylsulfatase gene with MET6 gene as a marker, primers SuCd-M6F and SuCd-M6R each having 40 bases of homologous sequence of arylsulfatase gene added to the 5'-side were designed. The homologous sequences of arylsulfatase gene were near the restriction enzyme sites for Cla I, which existed two locations in the open reading frame. As shown in Figure 4, a fragment having homologous sequences to arylsulfatase at the both ends of MET6 gene was obtained by using these primers and genomic DNA as the template. The obtained fragment was subcloned into pGEM (registered trademark) - T vector (produced by Promega) to obtain pdSuCM6. The various methods used for construction were similar to those stated above.

### (Transformation of L-methionine and L-histidine double nutrient-requiring mutant 8-23 strain by using a vector for disrupting arylsulfatase gene)

Figure 5 represents a schematic diagram of construction of a transformant. Plasmid pdSuC1 was linearized by treating with Nco I and Aat II, followed by transformation of the 8-23 strain with the linearized plasmid by the lithium acetate method. Thus, a transformant, SuCD strain, was obtained, which strain grew on SD medium with 20 µg/mL of methionine added.

Then, plasmid pdSuCM6 was linearized by treating with Cla I, followed by transformation of the SuCD strain with the linearized plasmid by the lithium acetate method. Thus, a transformant, SuCDD5-2 strain, was obtained, which strain grew on SD medium.

### (Southern blotting of DNAs from parent strain and transformant strains)

Obtained transformants were respectively cultured in YPD medium, and genomic DNAs were respectively prepared from the culture fluids using Dr. GenTLE™ (from yeast) (produced by Takara Bio Inc.). The obtained genomic DNAs were digested with Bam HI, followed by Southern analyses. As the probe, the Aat II-Eco RI fragment of arylsulfatase gene was used. For labeling and detection of nucleic acid, AlkPhos Direct Labeling and Detection System with CDP-Star (produced by GE Healthcare Bioscience Co., Ltd.) was used, and the method of using was according to the attached document.

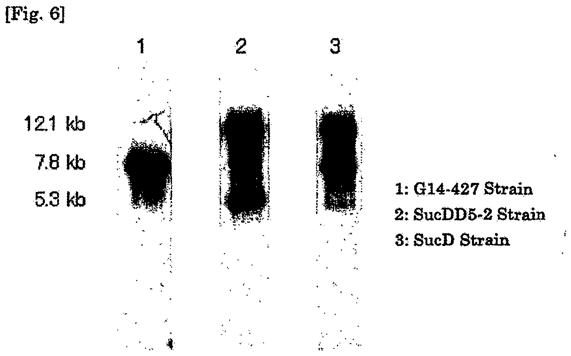

The results of Southern blotting are shown in Figure 6. Lanes 1, 2, and 3 represent the parent strain G14-427, the transformant SuCDD5-2, and the transformant SuCD, respectively. In lane 3, with the band (12.1kb) of fragment containing arylsulfatase gene and HIS4 gene, a band at the same position (7.8 kb) in lane 1 was also detected. Thus, it was confirmed that only one of the arylsulfatase genes had been disrupted. On the other hand, in lane 2, the band of 7.8 kb shifted to 5.3 kb. Thus, it was confirmed that both arylsulfatase genes had been disrupted.

### (Detection of arylsulfatase activity in arylsulfatase gene-double disrupted strain)

The parent strain, Kluyveromyces lactis G14-427 strain (a diploid strain) and the SuCDD5-2 strain constructed as described above were respectively inoculated to YPD medium, and incubated at 30 degrees Celsius, with shaking at 210rpm for 72 hours. To each culture fluid, Zymoliase (produced by Seikagaku Biobusiness Corp.) was added to be a concentration of 2 mg/mL, and reaction was performed at 30 degrees Celsius for 2 hours to disrupt the cell walls. Supernatants were respectively gathered by centrifugation, and the lactase activity and arylsulfatase activity were determined by the FCC IV method and the method as described in Example 7, respectively. The results are shown in Table 12.

It has been revealed that although in the culture fluid of the SuCDD5-2 strain, lactase is contained, arylsulfatase is not contained or is contained in a very small amount if any such that it cannot be detected by the fluorescence method. Namely, it has been confirmed that although the SuCDD5-2 strain maintains the productivity of lactase, productivity of arylsulfatase is zero or almost zero.

**[Table 12]**

| Comparison between Arylsulfatase Activity of Strain having Two Arylsulfatase Genes and Other Strain in Which Both Arylsulfatase Genes Had Been Disrupted | | |
|---|---|---|
| Strain that Produces Lactase | Lactase Activity (NLU/g) | Arylsulfatase Activity (U/g) |
| G14-427 Strain | 16 | 6 |
| SucDD5-2 Strain | 16 | 0 |

### [Example 12] Preparation of enzyme preparations with SF-81 strain and CBS2359 strain

The SF-81 strain (a diploid mutant with reduced arylsulfatase productivity) obtained in Example 9 and CBS2359 strain (a monoploid strain) were respectively inoculated to a medium for lactase production containing 7% corn steep liquor and 2% lactose, and incubated at 30 degrees Celsius with shaking at 210rpm for 96 hours. Then, the cells were respectively gathered by centrifugation. Sterile purified water was added to the cells and the cell walls of the gathered cells were disrupted with glass beads and ultrasonic waves. The thus obtained mixture containing cells, purified water, and the like, was centrifuged and supernatant was gathered. The lactase activity of the obtained supernatant was determined by the fluorescence method as described in Example 7. As a result, the relative activity of the CBS2359 strain was 2% as compared to the lactase activity of the SF-81 strain that was specified as 100%.

The above supernatant was fractionated with ammonium sulfate and concentrated with an ultrafiltration membrane. As a result, from cells of the SF-81 strain, lactase preparations were obtained, which preparations have respective lactase activity of about 1,000, 2,000, 3,000, 4,000, 5,000, or 6,000 NLU/g depending on the degree of concentration. On the other hand, no preparation was obtained having lactase activity of 1,000 NLU/g or higher from the CBS2359 strain although a similar concentration method was performed.

### [Example 13] Preparation of enzyme preparation

The SF-81 strain was inoculated to a medium for lactase production containing 7% corn steep liquor and 2% lactose, and incubated at 30 degrees Celsius with shaking at 210rpm for 96 hours. Then, the cells were gathered by centrifugation. Sterile purified water was added to the cells and the cell walls of the gathered cells were disrupted with glass beads and ultrasonic waves. The supernatant was gathered by centrifugation. The supernatant was fractionated with ammonium sulfate and concentrated by an ultrafiltration membrane to obtain a lactase preparation having lactase activity of about 5,000 NLU/g. The arylsulfatase activity of this lactase preparation was 1 U/g or less according to the method of the present invention (the fluorescence method).

### [Example 14] Organoleptic examination for taste (Part 2)

### (Preparation of lactase preparations with various contamination rates of arylsulfatase)

Five lactase preparations each having arylsulfatase activity of 1 to 20 U/g as determined by the fluorescence method shown in Example 7 were prepared from the lactase preparation per se produced from the SF-81 strain by the method as described in Example 13, and by appropriately mixing the lactase preparation with GODO-YNL2 (liquid lactase preparation, produced by Godo Shusei Co. Ltd.). Further, the lactase activity of each lactase preparation was determined by the FCC IV method.

### (Organoleptic examination for taste)

As is in Example 8, each of the 5 lactase preparations was added to a commercially available cow milk to be the lactase content of 20,000 NLU/L-milk, and the obtained cow milks were stored at 30 degrees Celsius. After 1 month of storage, an organoleptic examination for taste was performed by a similar method as described in Example 8, in which the milks to which lactase preparations had been respectively added were compared to the milk to which lactase preparation had not been added. The results are shown in Table 13.

**[Table 13]**

| Results of Organoleptic Examination for Taste (Off-flavor) of Cow Milks that Were Treated by Lactase Preparations Containing Arylsulfatase in Various Contamination Rates | | | |
|---|---|---|---|
| Lactase Preparations | | | Off-flavor |
| Lactase Activity (NLU/g) | Arylsulfatase Activity (U/g) | Arylsulfatase Activity / Lactase Activity (%) | Reaction Time of Period: 1 month |
| Not Added | | | - |
| 5000 | 1 or less | 0.02 or less | - |
| 5000 | 5 | 0.1 | - |
| 5000 | 10 | 0.2 | + |
| 5000 | 15 | 0.3 | + |
| 5000 | 20 | 0.4 | + |

| | | | |
|---|---|---|---|
| +: Aware -: Not Aware | | | |

From the results shown in Table 13, it has been concluded that arylsulfatase activity as determined by the method according to the present invention is preferably 5 U/g or less. Further, it has been demonstrated that the proportion of arylsulfatase activity (unit: U/g) as determined by the method according to the present invention is preferably 0.1% or less by using the lactase activity (unit: NLU/g) by the FCC IV method as the basis.

## Claims

1. A method for determining activity of arylsulfatase in an aqueous system **characterized in that** arylsulfatase that is derived from yeast is subjected to reaction with a substrate, from which fluorophore is liberated by suffering an action of the arylsulfatase, in an aqueous reaction system having high ionic strength to which an inorganic salt has been added, wherein the concentration of the inorganic salt in the aqueous reaction system ranges from 50 to 500mM.

2. The method for determining activity of arylsulfatase in an aqueous system according to claim 1, wherein the means for reaction in an aqueous reaction system having high ionic strength is one in which the enzyme is subjected to reaction with a substrate in a buffer that does not denature the enzyme protein.

3. The method for determining activity of arylsulfatase in an aqueous system according to claim 2, wherein the concentration of the buffer is 50 to 200mM.

4. The method for determining activity of arylsulfatase in an aqueous system according to any one of claims 1 to 3, wherein the inorganic salt is at least one member selected from the group consisting potassium chloride, sodium chloride, and ammonium sulfate.

5. The method for determining activity of arylsulfatase in an aqueous system according to any one of claims 2 to 4, wherein the buffer is a phosphate buffer.

6. The method for determining activity of arylsulfatase in an aqueous system according to claim 1 comprising the following steps (1) to (10):
(1) a specimen in which the existence of the arylsulfatase is predicted is arbitrarily diluted with 100mM potassium phosphate buffer (pH6.5) comprising 0.5M potassium chloride to obtain a sample,
(2) an aqueous solution comprising potassium 4-methylumbelliferone sulfate in a concentration of 2mM is prepared,
(3) the sample and the aqueous potassium 4-methylumbelliferone sulfate solution are mixed with each other at a ratio of 1:1 (volume basis) and are reacted at 37 degrees Celsius for 3 hours,
(4) to the reacted solution, 0.1N aqueous sodium hydroxide solution having the same amount (volume basis) as that of the reacted solution is added to stop the reaction, thus obtaining a sample for determination,
(5) fluorescence intensity is determined at an excitation wavelength of 360nm and a fluorescence wavelength of 450nm,
(6) 4-methylumbelliferone is dissolved in 100mM potassium phosphate buffer (pH6.5) comprising 0.5M potassium chloride to obtain a solution having an appropriate concentration, 0.1N aqueous sodium hydroxide solution is added in a similar way as that in step (4), and fluorescence intensity is determined under the same conditions as those in step (5),
(7) from step (6), a calibration curve is prepared,
(8) from the fluorescence intensity that was determined in step (5) and the calibration curve that was prepared in step (7), the concentration of 4-methylumbelliferone of the sample for determination is calculated, and the calculated value is divided by 3, thus obtaining the concentration of the 4-methylumbelliferone in the case where the reaction time of period is 1 hour; further from the volume of the reacted solution, the amount of the 4-methylumbelliferone that was liberated by the reaction of one hour is calculated,
(9) because the amount of the 4-methylumbelliferone thus calculated is based on the amount of the specimen that was contained in the sample prepared in step (1), the calculated amount is converted to that of the 4-methylumbelliferone per 1g of the specimen, and
(10) when the amount of the 4-methylumbelliferone that was liberated per 1 hour of the time of period of the reaction of the substrate and the enzyme is 1nmole, it is defined as 1 unit(U), and the unit is shown as a unit amount per 1g of the specimen (i.e., an enzyme preparation), namely, "unit(U)/g".

7. The method for determining activity of arylsulfatase in an aqueous system according to any one of claims 1 to 6, which is a method for determining activity of arylsulfatase in a lactase preparation.

8. A lactase preparation produced by using, as a raw material, cultured yeast or microorganic cells and/or culture fluid of those cells, wherein the yeast or microorganic cells are those of a diploid strain of yeast having a lactase gene, in which expression of arylsulfatase protein is restricted, or a gene-recombinant microorganism in which a lactase gene of yeast has been transformed and expression of arylsulfatase protein is restricted, and prepared without a step for removing arylsulfatase, **characterized in that** the lactase preparation has a lactase activity of 4,000NLU/g or more according to the FCC IV method and has an arylsulfatase activity of 0.1% or less of the lactase activity as the basis, in which the arylsulfatase activity (unit: U/g) has been determined and calculated by the method of claim 6.

9. The lactase preparation according to claim 8, wherein the arylsulfatase activity (unit: U/g) is 0.02% or less of the lactase activity (unit: NLU/g) according to the FCC IV method as the basis.

10. A method for producing a lactase preparation **characterized by** culturing a diploid strain of yeast having a lactase gene, in which expression of arylsulfatase protein is restricted, or a gene-recombinant microorganism in which a lactase gene of yeast has been transformed and expression of arylsulfatase protein is restricted; gathering yeast or microorganic cells without destroying their cell walls, gathering culture fluid with yeast or microorganic cells after destruction of their cell walls, or gathering culture fluid without destroying cell walls; and preparing a lactase preparation having a lactase activity of 4,000 NLU/g or more according to the FCC IV method and an arylsulfatase activity of 0.1% or less of the lactase activity as the basis, in which the arylsulfatase activity (unit: U/g) has been determined and calculated by the method of claim 6, by using, as a raw material, the gathered yeast or microorganic cells and/or gathered culture fluid without a step for removing arylsulfatase.

## Patentansprüche

1. Verfahren zur Bestimmung der Aktivität von Arylsulfatase in einem wässrigen System, **dadurch gekennzeichnet, dass** aus Hefe gewonnene Arylsulfatase in einem wässrigen Reaktionssystem mit einer hohen Ionenstärke, dem ein anorganisches Salz zugesetzt wurde, mit einem Substrat, aus dem durch eine Wirkung der Arylsulfatase ein Fluorophor freigesetzt wird, umgesetzt wird, wobei die Konzentration des anorganischen Salzes in dem wässrigen Reaktionssystem im Bereich von 50 bis 500 mM liegt.

2. Verfahren zur Bestimmung der Aktivität von Arylsulfatase in einem wässrigen System nach Anspruch 1, wobei es sich bei dem Mittel für die Reaktion in einem wässrigen Reaktionssystem mit einer hohen Ionenstärke um ein Mittel handelt, in dem das Enzym in einem Puffer, der das Enzymprotein nicht denaturiert, mit einem Substrat umgesetzt wird.

3. Verfahren zur Bestimmung der Aktivität von Arylsulfatase in einem wässrigen System gemäß Anspruch 2, wobei die Pufferkonzentration 50 bis 200 mM beträgt.

4. Verfahren zur Bestimmung der Aktivität von Arylsulfatase in einem wässrigen System nach einem der Ansprüche 1 bis 3, wobei es sich bei dem anorganischen Salz um mindestens eines ausgewählt aus der Gruppe bestehend aus Kaliumchlorid, Natriumchlorid und Ammoniumsulfat handelt.

5. Verfahren zur Bestimmung der Aktivität von Arylsulfatase in einem wässrigen System nach einem der Ansprüche 2 bis 4, wobei es sich bei dem Puffer um einen Phosphatpuffer handelt.

6. Verfahren zur Bestimmung der Aktivität von Arylsulfatase in einem wässrigen System gemäß Anspruch 1, umfassend die folgenden Schritte (1) bis (10):
(1) eine Testprobe, in der das Vorhandensein der Arylsulfatase vermutet wird, wird mit 100 mM Kaliumphosphatpuffer (pH 6,5) umfassend 0,5 M Kaliumchlorid beliebig verdünnt, um eine Probe zu erhalten,
(2) eine wässrige Lösung umfassend Kalium-4-Methylumbelliferonsulfat in einer Konzentration von 2 mM wird zubereitet,
(3) die Probe und die wässrige Kalium-4-Methylumbelliferonsulfat-Lösung werden in einem Verhältnis von 1:1 (auf Grundlage der Volumina) miteinander gemischt und bei 37 °C für eine Dauer von 3 Stunden miteinander umgesetzt,
(4) zu der umgesetzten Lösung wird 0,1 N wässrige Natriumhydroxidlösung in derselben Menge (auf Grundlage der Volumina) wie die umgesetzte Lösung zugegeben, um die Reaktion zu beenden, wodurch eine Probe für die Bestimmung erhalten wird,
(5) die Fluoreszenzintensität wird bei einer Anregungswellenlänge von 360 nm und einer Fluoreszenzwellenlänge von 450 nm bestimmt,
(6) 4-Methylumbelliferon wird in 100 mM Kaliumphosphatpuffer (pH 6,5) umfassend 0,5 M Kaliumchlorid aufgelöst, um eine Lösung mit einer geeigneten Konzentration zu erhalten, 0,1 N wässrige Natriumhydroxidlösung wird auf ähnliche Weise wie in Schritt (4) zugegeben und die Fluoreszenzintensität wird unter denselben Bedingungen wie in Schritt (5) bestimmt,
(7) aus Schritt (6) wird eine Kalibrierkurve erstellt,
(8) aus der in Schritt (5) ermittelten Fluoreszenzintensität und der in Schritt (7) erstellten Kalibrierkurve wird die Konzentration von 4-Methylumbelliferon in der zu bestimmenden Probe berechnet und der berechnete Wert wird durch 3 geteilt, wodurch die Konzentration des 4-Methylumbelliferons für den Fall einer Reaktionsdauer von 1 Stunde erhalten wird; darüber hinaus wird aus dem Volumen der umgesetzten Lösung die Menge an 4-Methylumbelliferon berechnet, die bei der einstündigen Reaktion freigesetzt wurde,
(9) da die auf diese Weise berechnete Menge an 4-Methylumbelliferon auf der Menge der Testprobe basiert, die in der in Schritt (1) vorbereiteten Probe enthalten war, wird die berechnete Menge auf die Menge an 4-Methylumbelliferon pro 1 g der Testprobe umgerechnet, und
(10) wenn die pro 1 Stunde Reaktionsdauer bei der Umsetzung von Substrat und Enzym freigesetzte Menge an 4-Methylumbelliferon 1 nMol beträgt, dann entspricht dies laut Definition 1 Einheit (E) und die Einheit wird als eine Einheitsmenge pro 1 g Testprobe (d.h. einer Enzymzubereitung) nämlich in "Einheit (E)/g" angegeben.

7. Verfahren zur Bestimmung der Aktivität von Arylsulfatase in einem wässrigen System nach einem der Ansprüche 1 bis 6, wobei es sich um ein Verfahren zur Bestimmung der Aktivität von Arylsulfatase in einer Laktasezubereitung handelt.

8. Laktasezubereitung, hergestellt durch Anwendung, als Rohstoff, von Hefekulturen oder Mikrobenzellen und/oder Kulturflüssigkeit dieser Zellen, wobei es sich bei den Hefe- oder Mikrobenzellen um einen diploiden Hefestamm mit einem Laktasegen, in dem die Expression des Arylsulfatase-Proteins eingeschränkt ist, oder um einen rekombinanten Mikroorganismus handelt, in den ein Hefe-Laktasegen transformiert wurde und in dem die Expression des Arylsulfatase-Proteins eingeschränkt ist, und zubereitet ohne einen Schritt zur Entfernung von Arylsulfatase, **dadurch gekennzeichnet, dass** die Laktasezubereitung eine Laktaseaktivität von 4.000 NLU/g oder mehr gemäß dem FCC IV-Verfahren aufweist und eine Arylsulfatase-Aktivität von 0,1 % oder weniger der Laktaseaktivität der Basis aufweist, in der die Arylsulfatase-Aktivität (Einheit: E/g) nach dem Verfahren von Anspruch 6 bestimmt und berechnet wurde.

9. Laktasezubereitung nach Anspruch 8, wobei die Arylsulfatase-Aktivität (Einheit: E/g) nach dem FCC IV-Verfahren 0,02 % oder weniger im Vergleich zur Basis beträgt.

10. Verfahren zur Herstellung einer Laktasezubereitung, **gekennzeichnet durch** das Züchten eines diploiden Hefestamms mit einem Laktasegen, in dem die Expression des Arylsulfatase-Proteins eingeschränkt ist, oder eines rekombinanten Mikroorganismus, in den ein Hefe-Laktasegen transformiert wurde und in dem die Expression des Arylsulfatase-Proteins eingeschränkt ist; Gewinnung von Hefe- oder Mikrobenzellen ohne Zerstörung von deren Zellwänden, Gewinnung von Kulturflüssigkeit mit Hefe- oder Mikrobenzellen nach Zerstörung von deren Zellwänden oder Gewinnung von Kulturflüssigkeit ohne Zerstörung von Zellwänden; und Zubereitung einer Laktasezubereitung mit einer Laktaseaktivität von 4.000 NLU/g oder mehr gemäß dem FCC IV-Verfahren und einer Arylsulfatase-Aktivität von 0,1 % oder weniger der Laktaseaktivität der Basis aufweist, in der die Arylsulfatase-Aktivität (Einheit: E/g) nach dem Verfahren von Anspruch 6 bestimmt und berechnet wurde, **durch** Anwendung, als ein Rohstoff, der gewonnenen Hefe- oder Mikrobenzellen und/oder der gewonnenen Kulturflüssigkeit ohne einen Schritt zur Entfernung von Arylsulfatase.

## Revendications

1. Procédé pour déterminer une activité d'arylsulfatase dans un système aqueux **caractérisé en ce que** l'arylsulfatase qui est dérivée à partir de levure est soumise à une réaction avec un substrat, à partir duquel un fluorophore est libéré en subissant une action de l'arylsulfatase, dans un système de réaction aqueux possédant une force ionique élevée auquel un sel inorganique a été ajouté, dans lequel la concentration du sel inorganique dans le système de réaction aqueux varie entre 50 et 500 mM.

2. Procédé pour déterminer une activité d'arylsulfatase dans un système aqueux selon la revendication 1, dans lequel le moyen pour effectuer une réaction dans un système de réaction aqueux possédant une force unique élevée est un dans lequel l'enzyme est soumise à une réaction avec un substrat dans un tampon qui ne dénature pas la protéine enzymatique.

3. Procédé pour déterminer une activité d'arylsulfatase dans un système aqueux selon la revendication 2, dans lequel la concentration du tampon est comprise entre 50 et 200 nM.

4. Procédé pour déterminer une activité d'arylsulfatase dans un système aqueux selon l'une quelconque des revendications 1 à 3, dans lequel le sel inorganique est au moins un membre choisi parmi le groupe constitué par du chlorure de potassium, du chlorure de sodium et du sulfate d'ammonium.

5. Procédé pour déterminer une activité d'arylsulfatase dans un système aqueux selon l'une quelconque des revendications 2 à 4, dans lequel le tampon est un tampon phosphate.

6. Procédé pour déterminer une activité d'arylsulfatase dans un système aqueux selon la revendication 1 comprenant les étapes (1) à (10) suivantes :
(1) un spécimen dans lequel l'existence de l'arylsulfatase est prédite est arbitrairement dilué avec 100 mM de tampon phosphate de potassium (pH 6,5) comprenant 0,5 M de chlorure de potassium pour obtenir un échantillon,
(2) une solution aqueuse comprenant du sulfate de 4-méthylumbélliférone de potassium à une concentration de 2 mM est préparée,
(3) l'échantillon et la solution de sulfate de 4-méthylumbélliférone de potassium sont mélangés l'un avec l'autre dans un rapport de 1:1 (sur la base du volume) et sont mis en réaction à 37 °C pendant 3 heures,
(4) à la solution ayant réagi, 0,1 N d'une solution d'hydroxyde de sodium aqueux possédant la même quantité (sur la base du volume) que celle de la solution ayant réagi est ajoutée afin d'arrêter la réaction, obtenant ainsi un échantillon pour une détermination,
(5) une intensité de fluorescence est déterminée à une longueur d'onde d'excitation de 360 nm et à une longueur d'onde de fluorescence de 450 nm,
(6) de la 4-méthylumbélliférone est dissoute dans 100 nM de tampon de phosphate de potassium (pH 6,5) comprenant 0,5 M de chlorure de potassium pour obtenir une solution possédant une concentration adéquate, 0,1 N de solution d'hydroxyde de sodium aqueux est ajoutée d'une façon similaire que celle décrite lors de l'étape (4), et une intensité de fluorescence est déterminée dans les mêmes conditions que celles de l'étape (5),
(7) à partir de l'étape (6), une courbe de calibration est préparée,
(8) à partir de l'intensité de fluorescence qui a été déterminée lors de l'étape (5) et la courbe de calibration qui a été préparée lors de l'étape (7), la concentration de la 4-méthylumbélliférone de l'échantillon pour la détermination est calculée, et la valeur calculée est divisée par trois, obtenant ainsi la concentration de la 4-méthylumbélliférone dans le cas où le temps de réaction de la période est d'une heure ; de plus à partir du volume de la solution ayant réagi, la quantité de la 4-méthylumbélliférone qui a été libérée par la réaction d'une heure est calculée,
(9) parce que la quantité de la 4-méthylumbélliférone ainsi calculée est basée sur la quantité du spécimen qui était contenue dans l'échantillon préparé lors de l'étape (1), la quantité calculée est convertie en celle de la 4-méthylumbélliférone pour 1 g du spécimen, et
(10) lorsque la quantité de la 4-méthylumbélliférone qui a été libérée pendant 1 heure du temps de période de la réaction du substrat et de l'enzyme est de 1 nmole, elle est définie comme 1 unité (U), et l'unité est montrée comme une quantité d'unités pour 1 g du spécimen (c.-à-d., une préparation enzymatique), à savoir, "unité (U)/g".

7. Procédé pour déterminer une activité d'arylsulfatase dans un système aqueux selon l'une quelconque des revendications 1 à 6, qui est un procédé pour déterminer l'activité d'arylsulfatase dans une préparation de lactase.

8. Préparation de lactase produite en utilisant, en tant que matière première, de la levure cultivée ou des cellules microorganiques et/ou un fluide de culture de ces cellules, dans laquelle la levure ou les cellules microorganiques sont celles d'une souche diploïde de levure possédant un gène lactase, dans laquelle une expression de la protéine arylsulfatase est restreinte, ou un micro-organisme recombinant les gènes dans lequel un gène lactase de levure a été transformé et une expression de la protéine arylsulfatase est restreinte, et préparée sans nécessiter une étape d'élimination d'arylsulfatase, **caractérisée en ce que** la préparation de lactase possède une activité de lactase de 4000 NLU/g ou plus selon le procédé FCC IV et possède une activité d'arylsulfatase de 0,1 % au moins de l'activité de lactase en tant que base, dans laquelle l'activité d'arylsulfatase (unité : U/g) a été déterminée et calculée selon le procédé de la revendication 6.

9. Préparation de lactase selon la revendication 8, dans laquelle l'activité d'arylsulfatase (unité : U/g) est de 0,02 % ou moins de l'activité de lactase (unité : NLU/g) selon le procédé FCC IV comme base.

10. Procédé de production d'une préparation de lactase **caractérisé par** la culture d'une souche diploïde de levure possédant un gène de lactase, dans lequel une expression de la protéine arylsulfatase est restreinte, ou un micro-organisme recombinant les gènes dans lequel un gène de lactase de levure a été transformé et une expression de la protéine arylsulfatase est restreinte ; la collection de levure ou de cellules microorganiques sans détruire leurs parois cellulaires, la collection de fluide de culture avec une levure ou des cellules microorganiques après la destruction de leurs parois cellulaires, ou la collection de fluide de culture sans détruire les parois cellulaires ; et la préparation d'une préparation de lactase possédant une activité de lactase de 4000 NLU/g ou moins selon le procédé FCC IV et une activité d'arylsulfatase de 0,1 % ou moins de l'activité de lactase comme base, dans lequel l'activité d'arylsulfatase (unité : U/g) a été déterminée et calculée par le procédé selon la revendication 6, en utilisant, en tant que matière première, la levure collectée ou les cellules microorganiques collectées et/ou du fluide de culture collecté sans nécessiter une étape pour éliminer l'arylsulfatase.
